Europäisches Patentamt

European Patent Office

Office européen des brevets

(11) Publication number: **0 497 021 A1**

# EUROPEAN PATENT APPLICATION

(21) Application number: **91300749.8**

(22) Date of filing: **31.01.91**

(51) Int. Cl.5: **A61B 5/00**

(43) Date of publication of application:
**05.08.92 Bulletin 92/32**

(84) Designated Contracting States:
**DE FR GB**

(71) Applicant: **HAMAMATSU PHOTONICS K.K.**
**1126-1 Ichino-cho Hamamatsu-shi**
**Shizuoka-ken(JP)**

(72) Inventor: **Delpy, David Thomas, University**

College London
Med.Phys.& Bio-Eng., 1st Floor Shropshire
House
11-20 Capper Street, London WC1E 6JA(GB)

(74) Representative: **Rackham, Stephen Neil et al**
**GILL JENNINGS & EVERY 53-64 Chancery**
**Lane**
**London WC2A 1HN(GB)**

(54) **Oximeter with monitor.**

(57) A diagnostic apparatus includes light sources (LD1 to LD4) emit four near infrared light rays of different wavelengths. The light rays are introduced into a body organ to be subjected to diagnosis and the light rays transilluminated through the body organ are picked up. The transilluminated light is analysed to provide variations in concentration of both oxyhaemoglobin and deoxyhaemoglobin, $\Delta[X_{HbO2}]$ and $\Delta[X_{Hb}]$, in the organ. A CPU (64) provided in the apparatus performs an arithmetic operation of

$$K \times \Delta[X_{HbO2}] / \{\Delta[X_{Hb}] + \Delta[X_{HbO2}]\}$$

and provides an operation result, where K is a predetermined constant. The operation result is displayed on a display (64).

FIG. 1

The present invention relates generally to a oximeter, and more particularly to a oximeter with a monitor for displaying an oxygen saturation rate in objects, such as body organs, e.g., the cerebral tissues of a human body.

In general, in diagnosing the function of a body organ, such as the cerebral tissues, the fundamental and important parameters to measure are the oxygen quantity in the body organ and the organ's utilization of oxygen. Supplying body organs with a sufficient quantity of oxygen is indispensable for the growth ability of foetuses and new-born infants. If the supply of oxygen to a foetus is insufficient, the probability that the foetus will not survive or that the new-born infant will die is high. Even if the newborn infant lives, serious problems in the body organs may remain as sequels. The insufficiency of oxygen affects every body organ, but especially causes a serious damage in the cerebral tissues.

In view of the foregoing, it has been required to provide a diagnostic apparatus capable of measuring the status of an oxygen quantity in the cerebral tissues. Conventional diagnostic apparatuses are capable of measuring the variation of oxygen quantity in the brain through the absorption spectrum of near infrared light. The absorption is caused by the haemoglobin which is an oxygen-carrying medium in blood and the cytochrome a, $a_3$ which performs oxidation-reduction reaction in cells. In the conventional apparatuses, the amounts of oxyhaemoglobin ($HbO_2$), deoxyhaemoglobin (Hb), and the sum or subtraction of the amounts of both oxyhaemoglobin and deoxyhaemoglobin can be measured and displayed for diagnosis purpose.

However, a oxygen saturation rate of haemoglobin at a time when a cerebral blood volume has changed is not available in the conventional apparatus notwithstanding the fact that such information is useful in the diagnosis procedure.

According to this invention a diagnostic apparatus comprising:

light source means for sequentially emitting light of different wavelength;

means for introducing the light into an organ to be subjected to diagnosis and picking up light transilluminated through the organ;

means for analysing the transilluminated light and providing an operation result, and,

means for displaying the operation result;

is characterised in that the means for analysing the transilluminated light provides variations in concentration of oxygenated media $\Delta X_{02}$ and disoxygenated media $\Delta X$ in the organ; and, includes

means for performing an arithmetic operation of

$$K \times \Delta X_{02}/\{\Delta X + \Delta X_{02}\},$$

where K is a predetermined constant.

Accordingly, the present invention provides a diagnostic apparatus in which the oxygen saturation rate of haemoglobin of a changed cerebral blood volume can be automatically computed with the resultant data being displayed on a display unit.

In this invention, the lights introduced into the organ are near infrared lights in the case where the oxygenated media is oxyhaemoglobin ($HbO_2$) and the deoxygenated media is deoxyhaemoglobin (Hb).

In this apparatus, the lights with different wavelengths are repeatedly introduced into the organs each for a predetermined period of time, and the arithmetic operation is performed when a value of $|\Delta X + \Delta X_{02}|$ is equal to or larger than a predetermined minimum.

The invention will be better understood from the following description, given by way of example with reference to the accompanying drawings in which:

Fig. 1 is a system constitution of a diagnostic apparatus;

Figs. 2(a) through 2(e) are timing charts of driving signals ACT1 to ACT4 and a control signal CTL, respectively;

Figs. 3(a) and 3(b) are graphs of absorption spectra of haemoglobin and cytochrome, respectively;

Fig. 4 is a flow chart for description of the arithmetic operation performed by a CPU in the diagnostic apparatus; and

Figs. 5(a) and 5(b) are explanatory diagrams for description of the data to be obtained.

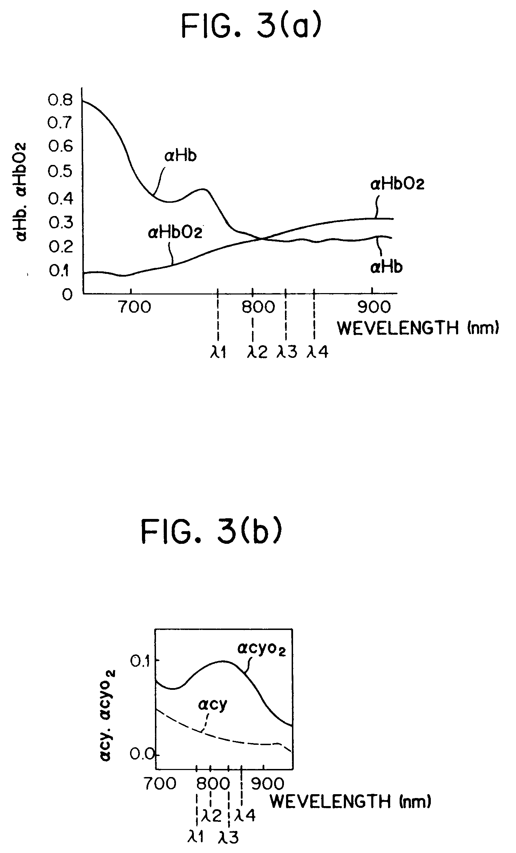

As shown in Fig. 3(a), the absorption spectra of near infrared light (700 to 1300 nm), $\alpha Hb_2$ and a Hb by oxyhaemoglobin ($HbO_2$) and deoxyhaemoglobin (Hb), respectively, are different from each other. And as shown in Fig. 3(b), the absorption spectra of $\alpha CyO_2$ and $\alpha Cy$ by oxidized cytochrome a, $a_3$ (Cy), respectively, are different from each other. This diagnostic apparatus utilizes the above-described absorption spectra of near infrared light. Four near infrared light rays with different wavelengths, $\lambda_1, \lambda_2, \lambda_3, \lambda_4$ (e.g. 775nm, 800nm, 825nm, and 850nm) are applied to one side of the patient's head with a time-sharing method and the transmission light rays from the opposite side of the head are in turn detected. By processing these four detected light rays with the prescribed calculation program, the density variations of

2

oxyhaemoglobin ($HbO_2$), deoxyhaemoglobin (Hb), oxidized cytochrome a, $a_3$ ($CyO_2$) and reduced cytochrome a, $a_3$ (Cy) are calculated. These parameters, in turn, determine the variation of cerebral oxygen quantity.

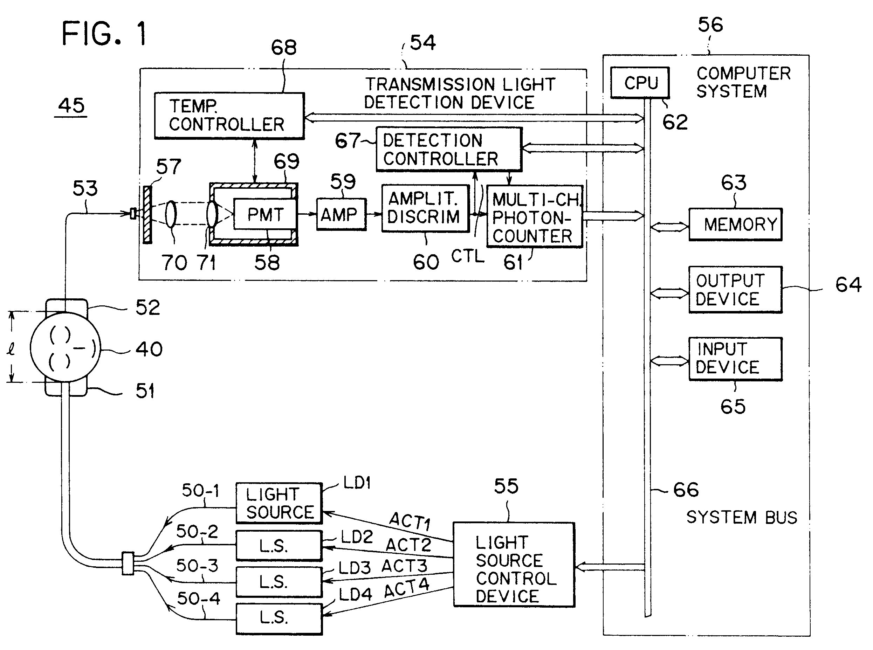

Fig. 1 shows a system outline of a diagnostic apparatus 45. The apparatus 45 includes light sources such as laser diodes LD1 to LD4 which emit four near infrared light rays with different wavelengths of $\lambda_1$, $\lambda_2$, $\lambda_3$ and $\lambda_4$ respectively; a light source control device 55 which controls output timing of the light sources LD1 to LD4; optical fibers 50-1 to 50-4 which introduces near infrared light rays emitted by the light sources LD1 to LD4 to a patient's head 40; an illumination-side fixture 51 which bundles and holds end portions of the optical fibers 50-1 to 50-4; a detection-side fixture 52 which is fitted to the prescribed position of the opposite side of the patient's head 40; an optical fiber 53 which is held by the detection-side fixture 52 and introduces transmitted near infrared light from the patient's head 40; a transmission light detection device 54 which measures transmission quantity of near infrared light by counting photons of near infrared light introduced by the optical fiber 53; and a computer system 56 which controls the total diagnostic apparatus and determines the variation of oxygen quantity in cerebral tissues based on the transmission quantity of near infrared light.

The computer system 56 is equipped with a central processing unit (CPU) 62, a memory 63, output devices 64 such as a display and printer, and an input device 65 such as a keyboard, and these devices are connected to each other by a system bus 66. The light source control device 55 and the transmission light detection device 54 are connected to the system bus 66 as external I/O's.

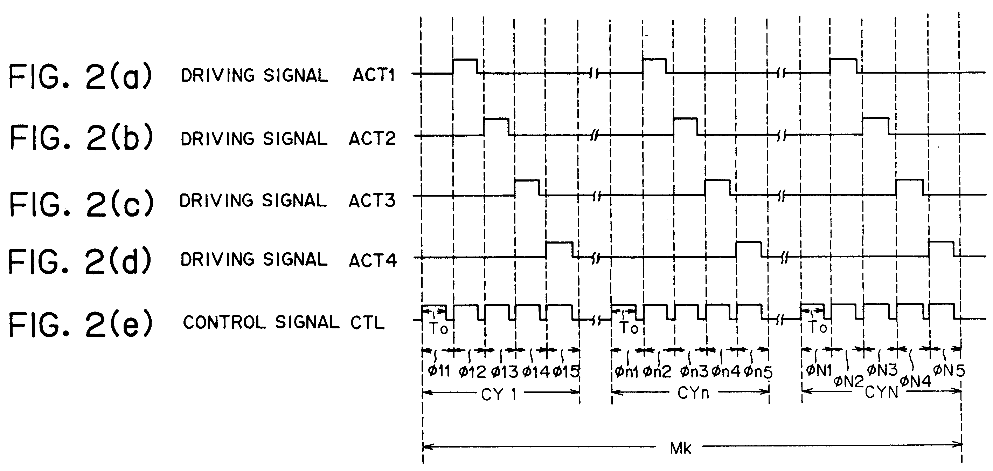

The light source control device 55 receives instructions from the computer system 56 and drives the light sources LD1 to LD4 by respective driving signals ACT1 to ACT4 as shown in Figs. 2(a) to 2(d). As shown therein, one measuring period $M_k$ (k = 1, 2,...) consists of N cycles of CY1 to CYn. At a phase $\phi$ nl in an arbitrary cycle CYn, no light source of LD1 to LD4 is driven and therefore the patient's head 40 is not illuminated by the near infrared light from the light sources LD1 to LD4. At the phase $\phi$ n2 the light source LD1 is driven and the near infrared light with the wavelength of, for example, 775 nm is emitted from it. In the same manner, at the phase $\phi$ n3 the light source LD2 is driven and the near infrared light with the wavelength of, for example, 800 nm is emitted from it; at the phase $\phi$ n4 the light source LD3 is driven and the near infrared light with the wavelength of, for example, 825 nm is emitted from it; and at the phase $\phi$ n5 the light source LD4 is driven and the near infrared light with the wavelength of, for example, 850 nm is emitted from it. In this manner, the light source control device 55 drives the light sources LD1 to LD4 sequentially with a time-sharing method.

Referring again to Fig.1, the transmission light detection device 54 is equipped with a filter 57 which adjusts the quantity of near infrared light outputted to lenses 70 and 71 from the optical fiber 53; a photomultiplier tube 58 which converts the light from the filter 57 into pulse current and outputs it; an amplifier 59 which amplifies the pulse current from the photo multiplier tube 58; an amplitude discriminator 60 which eliminates the pulse current from the amplifier 59 whose amplitude is smaller than the prescribed threshold value; a multichannel photoncounter 61 which detects photon frequency in every channel; a detection controller 68 which controls the temperature of a cooler 69 containing the photomultiplier tube 58.

To use the above-described diagnostic apparatus, the illumination-side fixture 51 and the detection-side fixture 52 are firmly fitted to the prescribed positions of the patient's head 40 by using tape or the like. Once fitted, the light sources LD1 to LD4 are driven by the light source control device 55 as shown in Figs. 2(a) to 2(d), respectively, so that the four near infrared light rays with different wavelengths are emitted from the light sources LD1 to LD4 sequentially with the time-sharing method, and the light rays are introduced by the optical fibers 50-1 to 50-4 to the patient's head 40. As bones and soft tissues in the patient's head 40 are transparent to the near infrared light, the near infrared light is partially absorbed by haemoglobin in blood and cytochrome a, a3 in cells and out-putted to the optical fiber 53. The optical fiber 53 introduces the light to the transmission light detection device 54. At the phase $\phi$ nl, no light source of LD1 to LD4 is driven, and therefore, the transmission light detection device 54 detects dark light.

The photomultiplier tube 58 in the transmission light detection device 54 is used with a photon-counting device that has high sensitivity and operates at high response speed. The output pulse current from the photomultiplier tube 58 is sent to the amplitude discriminator 60 through the amplifier 59. The amplitude discriminator 60 eliminates the noise component whose amplitude is smaller than the prescribed amplitude threshold and sends only the signal pulse to the multi-channel photon-counter 61. The multi-channel photon-counter 61 detects photons only in the periods $T_0$. The periods $T_0$ are synchronized with the driving signals ACT1 to ACT4 for the respective light sources LD1 to LD4 as shown in Fig. 2(a) to 2(d) by a control signal CTL as shown in Fig. 2(e). The control signal CTL is generated by the detection controller 67. The multi-channel photo-counter then counts detected photon number of every light with each wavelength sent from the optical fiber 53. The transmission data of every infrared light with each wavelength are obtained through the above-described procedure.

As shown in Figs. 2(a) to 2(e), at the phase $\phi$ nl in the cycle CYn of light source control device 55 no light source of LD1 to LD4 is driven, therefore the dark light data d are counted by the transmission light detection device 54. At the phases $\phi$ n2 to $\phi$ n5 the light sources LD1 to LD4 are sequentially driven with the time-sharing method and the transmission light detection device 54 sequentially counts the transmission data $t_{\lambda 1}$, $t_{\lambda 2}$, $t_{\lambda 3}$ and $t_{\lambda 4}$ of the respective near infrared light rays with different wavelengths $\lambda$ 1, $\lambda$ 2, $\lambda$ 3 and $\lambda$ 4.

The counting of the dart light data d and the transmission data $t_{\lambda 1}$, $t_{\lambda 2}$, $t_{\lambda 3}$ and $t_{\lambda 4}$ which is sequentially performed in the cycle CYn, is continued N times from CY1 to CYn. That is, one measuring period $M_k$ ($k = 1,2,...$)includes N cycles. A concrete example is as follows; if one cycle is 200 $\mu$ sec and N is 10000, the measuring period $M_k$ becomes 2 sec. At the time of finishing of one measuring period $M_k$,the counting result of the dark light data D

$$\left[ = \sum_{n=1}^{N} d/CYn \right]$$

and the counting results of the transmission data $T_{\lambda 1}$,$T_{\lambda 2}$, $T_{\lambda 3}$ and $T_{\lambda 4}$

$$\left[ = \sum_{n=1}^{N} t\lambda_j/CYn \right]$$

are transferred to the computer system 56 and stored in the memory 63.

The processor 62 performs the subtraction of the dark light component by using the combination of the transmission data and the dark data $(T_{\lambda 1}, T_{\lambda 2}, T_{\lambda 3}, T_{\lambda 4}, D)_{Mk}$ being stored in the memory 63 after one measuring period $M_k$ and the combination of those $(T_{\lambda 1}, T_{\lambda 2}, T_{\lambda 3}, T_{\lambda 4}, D)_{Mo}$ at the start of measuring, and calculates the variation rates of the transmission light $\Delta T_{\lambda 1}$, $\Delta T_{\lambda 2}$, $\Delta T_{\lambda 3}$ and $\Delta T_{\lambda 4}$. That is, the variation rates of the transmission light $\Delta T_{\lambda 1}$, $\Delta T_{\lambda 2}$, $\Delta T_{\lambda 3}$, and $\Delta T_{\lambda 4}$ are calculated as:

$$\Delta T_{\lambda j} = \log [(T_{\lambda j} - D)_{MK} / (T_{\lambda j} - D)_{MO}] \quad (j = 1, \text{ to } 4) \quad (1)$$

The use of logarithm in the above calculation of $\Delta T_{\lambda j}$ is to express the variation as an optical density.

Using the above-calculated variation rates of the transmission light $\Delta T_{\lambda 1}$, $\Delta T_{\lambda 2}$, $\Delta T_{1\lambda 3}$ and $\Delta T_{\lambda 4}$, density variations of oxyhaemoglobin (HbO2), deoxyhaemoglobin (Hb), oxidized cytochrome a, $a_3$ (CyO$_2$) and reduced cytochrome a, $a_3$ which are expressed as $\Delta X_{HbO2}$ $\Delta X_{Hb}$, $\Delta X_{CyO2}$ and $\Delta_{Cy}$, respectively, can be determined. That is, each of density variations of $\Delta X_{HbO2}$ $\Delta X_{Hb}$, $\Delta X_{CyO2}$ and $\Delta X_{Cy}$ is calculated as:

$$(2)$$

$$\Delta X_i = \sum_{j=1}^{4} (\alpha ij) - {}^1\Delta T_{\lambda i}/\ell$$

where $\alpha_{ij}$ is an absorption coefficient of each component i(HbO,Hb,CyO$_2$,Cy) for each wavelength $\lambda_j$($\lambda_1$, $\lambda_2$, $\lambda_3$, $\lambda_4$) and is predetermined from Figs. 3(a) and 3(b), and $\ell$ is the length of the patient's head 40 along the traveling direction of the near infrared light.

As the above-detected density variation components, $\Delta X_{HbO2}$, $\lambda X_{Hb}$, $\lambda X_{CyO2}$ and $\Delta X_{Cy}$, reflect the variation of oxygen quantity in the brain, the variation of oxygen quantity in the brain can be determined by outputting these detected results from the output device 64. The diagnosis thus is made based on these results.

The above-described arrangement of the diagnostic apparatus 45 is disclosed, for example, in U.S. Patent No. 4,901,238 issued February 13, 1990. In this apparatus, although it is possible to measure and display the density variations of oxyhaemoglobin (HbO$_2$), deoxyhaemoglobin (Hb), and oxidized cytochrome

4

(CyO$_2$) and reduced cytochrome (Cy), an oxygen saturation rate could not have been given. According to the present invention, the oxygen saturation rate is calculated based on the above-mentioned density variations and displays the resultant value in the output device 64.

The oxygen saturation rate of the increased or decreased blood $\Delta$SaO$_2$ to be obtained is defined by a ratio of the density variation of oxyhaemoglobin (HbO$_2$) to a sum of the density variations of oxyhaemoglobin (HbO$_2$) and deoxyhae- the density variations of oxyhaemoglobin (HbO$_2$) and deoxyhaemoglobin (Hb), i.e.,

$$\Delta SaO_2 = K \times \Delta[X_{HbO2}] / \{ \Delta[X_{Hb}] + \Delta[X_{HbO2}] \} \qquad (3)$$

where K is a predetermined constant, typically 100 for percentage representation.

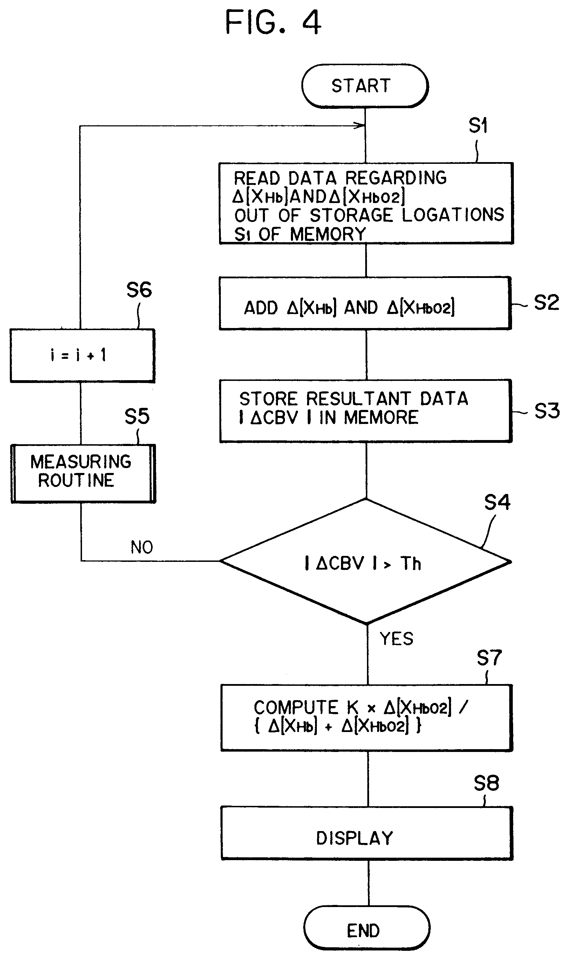

The arithmetic operation of the oxygen saturation rate is executed by the CPU 62 according to a sequence illustrated in the flow chart of Fig. 4. In the following description, assumption is made so that the data regarding $\Delta[X_{HbO2}]$ and $\Delta[X_{Hb}]$ measured during the period M$_i$ are sequentially stored in the storage location S$_i$ of the memory 63 upon completion of the measurement.

Referring to Fig. 4, the CPU 62 reads in step 1 the data regarding the $\Delta[X_{Hb}]$ and $\Delta[X_{HbO2}]$ out of the storage locations S$_i$ of the memory 63. Then, in step S2 the CPU 62 performs addition of $\Delta[X_{Hb}] + \Delta[X_{HbO2}]$, which corresponds to the computation of the denominator in equation (3). The computation executed in step S2 gives a variation in cerebral blood volume|$\Delta$CBV| in the tissue subjected to diagnosis. In step S3, the CPU 62 temporarily stores the data in relevant locations of the memory 63. Then, upon reading the data |$\Delta$CBV| out of the memory 63, the CPU 62 determines in step S4 if data |$\Delta$CBV| is greater than a threshold value Th. If no, the routine proceeds to step S5 where the measurement of the data $\Delta[X_{HbO2}]$ and $\Delta[X_{Hb}]$ in the subsequent measuring period M$_{i+1}$ is performed, whereupon the data obtained are stored in the storage location S$_{i+1}$ of the memory 63. In step S6, i is incremented by one so that the memory locations S$_{i+1}$ are specified and the processings in steps S1 through S4 are performed until the decision made in step S4 becomes YES. When the |$\Delta[CBV_{i+1}]$| is equal to or larger than the threshold value Th, then in step S7 the CPU performs arithmetic operation in accordance with equation (3) while utilizing the results obtained in step S2. In step S8, the result of the arithmetic operation is displayed in the output device 64.

As described, the lights with different wavelengths are repeatedly introduced into the organs for a predetermined period of time, and the arithmetic operation is performed when a value of |$\Delta[X_{HbO2}] + \Delta[X_{Hb}]$| is equal to or larger than a predetermined minimum to provide a reliable data in which the fluctuations of the measured data are substantially not involved.

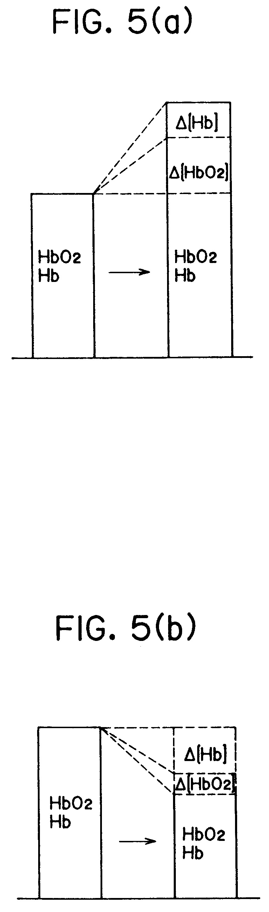

Fig. 5(a) shows the oxygen saturation ratio to be obtained in the case when the sum of the variations in concentration of the oxyhaemoglobin and deoxyhaemoglobin changes attendant to the increase of cerebral blood volume (CBV) in the tissue whereas Fig. 5(b) shows the oxygen saturation ratio in the case when the sum thereof changes attendant to the decrease of cerebral blood volume in the tissue.

## Claims

1. A diagnostic apparatus comprising:
    light source means (LD1,LD2,LD3,LD4,55) for sequentially emitting light of different wavelength;
    means (51,52) for introducing the light into an organ to be subjected to diagnosis and picking up light transilluminated through the organ;
    means (54,55) for analysing the transilluminated light and providing an operation result, and,
    means (64) for displaying the operation result;
    characterised in that the means (54,55) for analysing the transilluminated light provides variations in concentration of oxygenated media $\Delta X_{02}$ and disoxygenated media $\Delta X$ in the organ; and, includes means (62) for performing an arithmetic operation of

    $$K \times \Delta X_{02}/\{\Delta X + \Delta X_{02}\},$$

    where K is a predetermined constant.

2. A diagnostic apparatus according to claim 1, wherein the light introduced into the organ is near infrared light.

3. A diagnostic apparatus according to claim 1 or 2, wherein the light of different wavelength are repeatedly introduced into the organ each for a predetermined period of time, and wherein the arithmetic operation is performed when a value of $|\Delta X + \Delta X_{02}|$ is equal to or larger than a predetermined minimum.

4. A diagnostic apparatus according to any one of the preceding claims, wherein near infrared light of at least two different wavelength are introduced into the organ.

FIG. 1

FIG. 2(a)    DRIVING SIGNAL    ACT1

FIG. 2(b)    DRIVING SIGNAL    ACT2

FIG. 2(c)    DRIVING SIGNAL    ACT3

FIG. 2(d)    DRIVING SIGNAL    ACT4

FIG. 2(e)    CONTROL SIGNAL CTL

$\phi 11\ \phi 12\ \phi 13\ \phi 14\ \phi 15$    $T_0$    CY1

$\phi n1\ \phi n2\ \phi n3\ \phi n4\ \phi n5$    $T_0$    CYn

$\phi N1\ \phi N2\ \phi N3\ \phi N4\ \phi N5$    $T_0$    CYN

Mk

# FIG. 3(a)

# FIG. 3(b)

# FIG. 4

```
                    ┌─────────┐
                    │  START  │
                    └────┬────┘
                         │                              S1
                         ▼
            ┌────────────────────────────┐
            │ READ DATA REGARDING        │
            │ Δ[XHb] AND Δ[XHbO2]        │
            │ OUT OF STORAGE LOGATIONS   │
            │ Si OF MEMORY               │
            └────────────┬───────────────┘
                         │
      S6                 ▼                              S2
  ┌──────────┐  ┌────────────────────────────┐
  │ i = i + 1│  │ ADD Δ[XHb] AND Δ[XHbO2]    │
  └────┬─────┘  └────────────┬───────────────┘
       │                     │
  S5   │                     ▼                          S3
┌──────────────┐  ┌────────────────────────────┐
│ MEASURING    │  │ STORE RESULTANT DATA       │
│ ROUTINE      │  │ |ΔCBV| IN MEMORE           │
└──────┬───────┘  └────────────┬───────────────┘
       │                       │
       │                       ▼                        S4
       │    NO        ╱────────────────╲
       └──────────────┤  |ΔCBV| > Th    ├
                      ╲────────┬────────╱
                               │ YES
                               ▼                        S7
                  ┌────────────────────────────┐
                  │ COMPUTE K × Δ[XHbO2] /     │
                  │ { Δ[XHb] + Δ[XHbO2] }      │
                  └────────────┬───────────────┘
                               │
                               ▼                        S8
                  ┌────────────────────────────┐
                  │ DISPLAY                    │
                  └────────────┬───────────────┘
                               │
                               ▼
                          ┌─────────┐
                          │   END   │
                          └─────────┘
```

# FIG. 5(a)

Δ[Hb]

Δ[HbO₂]

HbO₂
Hb
→
HbO₂
Hb

# FIG. 5(b)

HbO₂
Hb
→
Δ[Hb]

Δ[HbO₂]

HbO₂
Hb

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | MEDICAL & BIOLOGICAL ENGINEERING & COMPUTING vol. 26, no. 3, 3 May 1988, pages 289-294, Stevenage, GB; M. COPE et al.: "System for long-term measurement of cerebal blood and tissue oxygenation on newborn infants by near infra-red transillumination"<br>* whole document; figures 1-3 * | 1 | A 61 B 5/00 |
| A | idem<br>--- | 2,4 | |
| Y | MEASURMENT TECHNIQUES vol. 32, no. 2, February 1989, pages 181-183, New York, US; A.G. BALASHOV et al., Biomedical Measurement: "Double-beam microelectronic instrument for nonivasive blood oxygen saturation measurement"<br>* pages 181, lines 1-16 *<br>--- | 1 | |
| A | US-A-4 714 341 (K. HAMAGURI et al.)<br>* abstract; figure 1,10d *<br>--- | 1,3 | **TECHNICAL FIELDS SEARCHED (Int. Cl.5)** |
| A | EP-A-0 290 278 (HAMAMATSU PHOTONICS K.K.)<br>* page 2, line 29 - page 4, line 13; figures 4,5,6 *<br>--- | 1,2,4 | A 61 B 5/00<br>G 01 N 21/31 |
| A | GB-A-2 075 668 (DUKE UNIVERSITY INC.)<br>* abstract; figures 4-6 *<br>----- | 1,2,4 | |

The present search report has been drawn up for all claims

| Place of search | Date of completion of the search | Examiner |
|---|---|---|
| BERLIN | 09-09-1991 | WEIHS J.A. |